Europäisches Patentamt

European Patent Office

Office européen des brevets

(11) Publication number: **0 149 510**
**B1**

(12) **EUROPEAN PATENT SPECIFICATION**

(45) Date of publication of patent specification: **22.03.89**

(21) Application number: **85300026.3**

(22) Date of filing: **03.01.85**

(51) Int. Cl.⁴: **A 61 D 7/00**

(54) **sustained release device for ruminants.**

(30) Priority: **06.01.84 US 568974**

(43) Date of publication of application:
**24.07.85 Bulletin 85/30**

(45) Publication of the grant of the patent:
**22.03.89 Bulletin 89/12**

(84) Designated Contracting States:
**AT BE CH DE FR GB IT LI LU NL SE**

(56) References cited:
**EP-A-0 062 391**
**EP-A-0 079 724**

(73) Proprietor: **ELI LILLY AND COMPANY**
**307, East McCarty Street**
**Indianapolis Indiana 46285 (US)**

(72) Inventor: **Roesch, Thomas Joseph**
**9613 Kelly Court**
**Indianapolis Indiana 46231 (US)**

(74) Representative: **Tapping, Kenneth George et al**
**Lilly Industries Limited Patent Department Erl**
**Wood Manor**
**Windlesham Surrey, GU20 6PH (GB)**

Courier Press, Leamington Spa, England.

## Description

This invention relates to a sustained release device for producing continuous administration of a biologically active agent or composition in the reticulorumen, of a ruminant animal, especially to provide for the controlled release of such an agent or composition over a prolonged period to range-fed ruminants, such as cattle or sheep.

US—A—4,251,506 discloses controlled-release compositions for administration of therapeutic agents, including weight-gain promoting agents. These compositions generally comprise one or more active agents embodied in a solid composition, such as a waxy carrier or surfactant. The agents may be dispersed throughout the composition either uniformly or in spaced layers. The patent proposes to enclose a solid plug or charge of the composition in a tubular capsule having a restricted opening at one end and a coil spring container at the other end to resiliently urge the plug or charge toward such opening. The capsule is administered into the reticulorumen, where the composition containing the active agent will be delivered or eroded into or absorbed by the ruminal fluid at the end opening of the capsule over an extended period. To retain the capsule in the rumen and prevent it from being regurgitated, the capsule of the patent is provided with a pair of resilient arms attached at an acute angle of approximately 45° to the body of the capsule at the delivery end thereof, and it is contemplated that these will be resiliently bent against the tubular body of the capsule when it is inserted through the esophagus of the animal to the rumen, and that such arms will then spring outward to stand at their normal acute angle of approximately 45°, like the barbs of an arrowhead, to retain the capsule in the rumen. Example 10 of the patent reports an experiment in which the growth stimulant monensin sodium was administered to Hereford heifers to produce a substantial weight gain over a trial period of sixty-three days. The patent discloses a capsule with its resilient arms at the open or delivery end of the capsule, but it is understood that the patentee has also used capsules on which the angular arms were attached to the closed end of the capsule opposite from the delivery end.

European Patent Application 62391 discloses a sustained release device for ruminants which has a steel ball at one end of the device to keep it in the rumen. However, it is necessary to keep the weighted device, or steel ball, from moving within its housing; and, in this European Patent Application disclosure, the means for doing this involves entrapment of the ball between an interior end and the actual drug that is to be dispensed on a sustained basis. Its drug release rate is dependent on a series of partitions instead of a spring and rumen fluid softening matrix concept.

It is an object of the present invention to provide an improved device and combination thereof with a medicament-containing charge which will provide more certain and longer lasting retention of the device in the reticulorumen, and will also facilitate oral administration of the device through the esophagus. It is a further object of the invention to provide better control of administration of the composition, with more uniform administration of the medicament over long periods.

EP—A—79724 discloses a two-part cylindrical capsule in which a solid drug matrix is held by spring action against an open end of the capsule, the capsule being retained in the reticulorumen by the action of resilient arms.

In accordance with the invention, the drug dispensing device utilizes a cylindrical housing made of plastic material and which has two sections that can be coupled together. One of these sections has a closed end and the other section has a pair of open ends, one of which is coupled to the first section. The other end of the first section has a drug restraining means such as a restricted opening through which a drug matrix dispersed upon coming into contact with the rumen fluid. The drug matrix is kept in constant sealing relationship with this restricted opening by a helical coil spring that is positioned between the closed end of the second section and a piston that is in contact with the drug. A cylindrical weight is maintained within this helical coil spring and has an annular groove that couples it to an annular retaining ring in the housing. Thus, the weight is trapped in a fixed position regardless of the amount of drug that remains in the capsule.

The present invention concerns a drug dispensing device for use in the reticulorumen of an animal comprising:

a cylindrical capsule formed of inert plastic material, said capsule having first and second hollow sections,

said first capsule section being of an elongated cylindrical configuration with a first open end and having a drug matrix restaining means,

said first section having a second open end opposite said first open end and formed with a coupling means,

said second capsule section having a closed end and an opposing open end with coupling means connecting it in sealed relationship to said first capsule section,

a cylindrical weight and a spring positioned within said second capsule,

a rumen fluid dissolvable or dispersible solid drug matrix of cylindrical configuration positioned within the interior of said capsule's first section,

said spring being biased to urge the distal end of said drug matrix against said drug matrix restraining means and toward said first capsule section's first open end, characterized in that said weight is encircled by said spring which is of helical configuration.

The accompanying drawing illustrates the invention and shows the preferred embodiment exemplifying the best mode of carrying out the invention as presently perceived. In such drawings:

Fig. 1 is a substantially full-scale longitudinal section of a capsule in accordance with the invention;

Fig. 2 is a perspective view of the piston in the capsule of Fig. 1;

Fig. 3 is a longitudinal section of Fig. 2;

Fig. 4 is a perspective view of a second embodiment of the capsule's piston; and

Fig. 5 is a longitudinal section of Fig. 4.

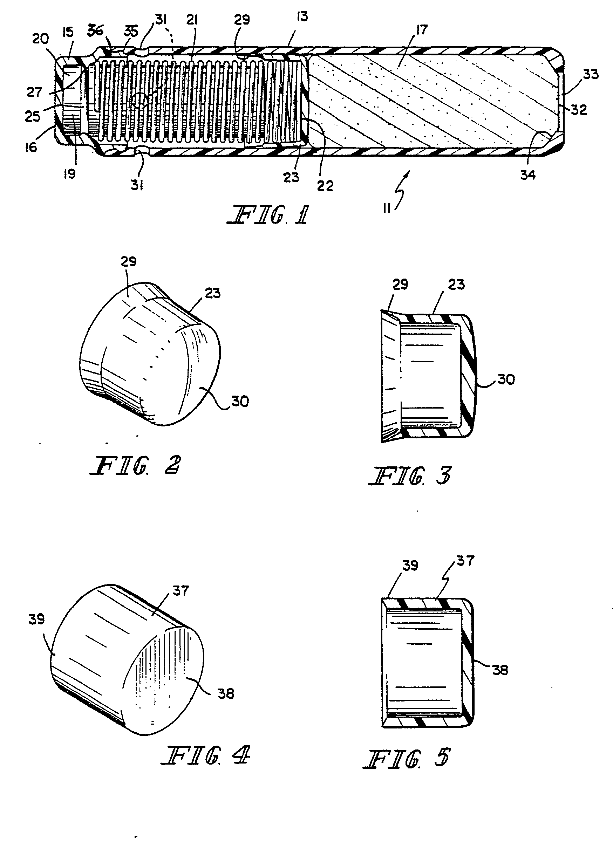

Referring to Fig. 1 of the drawing, capsule 11 is of cylindrical configuration and formed from an inert platic material such as polyethylene or polypropylene. It is to be noted that this capsule may be in the rumen of the animal for more than 200 days, and it is essential that it not be affected by the rumen fluid. Capsule 11 has a long first section 13 and a relatively short second section 15. A cylindrical shaped drug matrix 17 is contained within the first section and a cylindrical weight 19 occupies approximately the other half of the capsule. End 20 of weight 19 is preferably dimensioned to achieve a close fitting inside closed end 16 of section 15. A helical coil spring 21 encircles weight 19 and, at one end 22, abuts a piston 23. Weight 19 has a groove 25, which is in engagement with an annular ridge 27 that extends from the inner surface of section 15. Referring to Figs. 2 and 3, piston 23 can also be made of the same material as the capsule sections. Thus, piston 23 can be molded and has a relatively thin and outwardly extending skirt 29. The closed end of the piston has a contact surface 30 which is slightly convex.

The specific composition of drug matrix 17 will vary depending upon the nature of the active agent and in one embodiment may comprise the drug monensin sodium. The drug matrix is formed into a relatively solid but rumen fluid dispersible composition in order to administer the drug, to range animals over a prolonged period of approximately 200 days. The drug may also be many other substances such as an anthelmintic, other types of growth stimulants, vitamins and trace minerals. The end 32 of the drug matrix, adjacent opening 33 of the capsule, is exposed to ruminal temperature and fluid after the capsule is orally administered and resides in the animal's reticulorumen. This causes the solid composition of the drug matrix to weaken after which the active agent will be discharged into the ruminal fluid at a rate determined by controlling factors such as the nature of the composition and the configuration of opening 33. As the drug matrix 17 continues to disperse drug at opening 33, its length will be reduced. However, it is essential that the drug's end maintain its sealed relationship with the converging annular flange 34 that defines opening 33. Otherwise, rumen fluid will tend to seep down along the sides of the drug and create a different rate of delivery than can be maintained by limiting exposure to the area in opening 33. It is spring 21, in combination with piston 23, which maintains this sealing relationship. Spring 21 is in a compressed condition since its unstressed length will be at least twice the length of the capsule. Thus, the piston 23 is urged against drug matrix 17 by spring 21, and the exposed end of matrix 17 is continually forced against the annular flange 34 to effect a sealing relationship between them.

Keeping in mind that the animal, such as a steer, grazes on the rangelands and is quite active, it is essential that the capsule 13 be retained in the reticuloruman for the duration of drug delivery to avoid accidental displacement through regurgitation. It has been found that a minimum density of approximately 1.6 will enable a high percent of capsules of the described size to remain retained in the rumen of the animals. Weight 19, which is approximately $2\frac{1}{2}$ inches long, weighs 6.8 ounces (6.35 cm long, weighs 192.78 g). It is maintained in an immobile position within capsule 13 by the close fitting of its end 20 within closed end 16 of section 15 and an interior annular retaining ring or ridge 27 that interlocks with annular groove 25 of the weight. Its other end is maintained within piston 23. In this preferred embodiment the density is 2.2.

Piston 23 in the preferred embodiment has an outwardly extending tapered flange 29, which maintains a sealing relationship with the interior wall of capsule section 13. This is provided to prevent rumen fluid from entering through vents 31 and attacking portions other than face 32 of drug matrix 17. Four vents 31 have been provided in order to prevent the forming of negative pressure within section 13. Otherwise drug matrix 17, as it reduces in its length, would be creating a partial vacuum in the back side of section 13 and section 15 since it is snugly contained in the cylindrical wall of section 15. If such a partial vacuum is formed the payout rate of drug in matrix 17 could be affected to the extent of producing inconsistent results. Piston 23 is also provided with a convex head 30 in order to cope with possible bubble formation between it and the inner end of drug matrix 17.

Such bubbles may be formed as a result of silicone liquid that is first introduced into the capsule prior to loading it with drug matrix 17. This silicone enables the drug matrix 17 to maintain its capability of sliding against the inner surface of section 13. However, it has been found that on occasion the silicone liquid and air will become entrapped between a flat surfaced piston and the inner end of drug matrix 17. This can tend to have an adverse effect on the dispersing rate of the drug and is believed to result from a less than complete seal against flange 34 due to the variable compression effect of the bubbles. Domed surface 30 allows displacement of such bubbles.

Insertion of a capsule into the reticulorumen of a steer is conveniently done with an administration tool comprising a tubular barrel having an open end adapted to receive a capsule 11. The barrel of the tool is mounted on a tube having a handle portion by which the barrel can be manipulated through the mouth of the animal to place the end of the capsule 11 in the esophagus. The tube contains a thrust rod having a plunger at its forward end and a thrust knob at its outer end by which the plunger head can be advanced to discharge the capsule 11 from the barrel into the esophagus. When the weighted capsule is thus discharged partway into the esophagus, the capsule will then move on through the esophagus into the rumen. Tools of this general description have been used to insert boluses and capsules of other configurations and can readily be redesigned to apply capsule 11.

In assembling the capsule, approximately 5 cc of a lubricant such as silicone liquid is introduced into capsule section 13. Drug matrix 17 is then inserted through its larger open end until it is seated against flange 34. Piston 23 and spring 21 are then inserted into section 13 to butt against the inner end of drug matrix 17. Section 15 may then be snapped onto weight 19 and weight 19 is inserted into spring 21. This assembly is then coupled to section 13 by means of innerfitments 35 and 36. In addition, the capsule may be sonically welded at the junction line of sections 13 and 15. A removable label seal may be placed on the flanged end 34, preferably prior to adding the liquid lubricant. The capsule is now ready for use.

In the second embodiment of Figs. 4 and 5, piston 37 utilizes a flat surface or head 38 and a flat non-tapered wall 39. Consequently, it does not maintain a sealing relationship with the inner wall of section 13. Any bubbles initially entrapped between surface 38 and the drug will travel radially outward to the wall of section 13 and beyond the piston's wall 39.

## Claims

1. A drug dispensing device for use in the reticulorumen of an animal comprising:
a cylindrical capsule (11) formed of inert plastic material, said capsule having first (13) and second (15) hollow sections,
said first capsule section being of an elongated cylindrical configuration with a first open end (33) and having a drug matrix restraining means (34),
said first section having a second open end opposite said first open end and formed with a coupling means (35),
said second capsule section having a closed end (16) and an opposing open end with coupling means (36) connecting it in sealing relationship to said first capsule section,
a cylindrical weight (19) and a spring (21) positioned within said second capsule,
a rumen fluid dissolvable or dispersible solid drug matrix of cylindrical configuration (17) positioned within the interior of said capsule's first section,
said spring being biased to urge the distal end (32) of said drug matrix against said drug matrix restraining means and toward said first capsule section's first open end: characterized in that said weight is encircled by said spring which is of helical configuration.

2. A drug dispensing device in accordance with claim 1 in which said drug matrix restraining means (34) comprises a converging annular flange defining said first section's first opening.

3. A drug dispensing device in accordance with claim 2 in which said solid drug matrix is snugly contained in the cylindrical wall of said first capsule section.

4. A drug dispensing device in accordance with claim 3 in which said weight is immobilized within said capsule.

5. A drug dispensing device in accordance with claim 4 in which one end of said weight is in close fitment with the cylindrical wall of said second capsule section.

6. A drug dispensing device in accordance with claim 3 in which said cylindrical weight has circumferential portion (10) engaged with an interior retaining means (15) in said capsule.

7. A drug dispensing device in accordance with claim 6 in which the second section of said capsule has an annular retaining ring (27) which is seated in an annular groove (25) of said cylindrical weight.

8. A drug dispensing device in accordance with claim 7 in whiich a piston (23) is positioned between said drug matrix and the adjacent end (22) of said spring with a cylindrical side wall extending over said adjacent spring end and in close sliding contact with the interior wall of said capsule.

9. A drug dispensing device in accordance with claim 8 in which said piston has an outwardly extending tapered flange (29) in sealing relationship with said first section's cylindrical wall and in which said section has a vent hole.

## Patentansprüche

1. Medikamentenabgabevorrichtung zur Verwendung im Netzmagen eines Tieres, enthaltend:
eine zylindrische Kapsel (11) aus einem inerten Plastikmaterial, die erste (13) und zweite (15) hohle Sektionen aufweist,
wobei die erste Kapselsektion von langgestreckter, zylindrischer Gestalt ist mit einem ersten offenen Ende (33) und einer Medikamentenmatrixhalteeinrichtung (34),
wobei die erste Sektion ein zweites offenes Ende entgegensetzt zum ersten offenen Ende aufweist und mit einer Kupplungseinrichtung (35) versehen ist,
wobei die zweite Kapselsektion ein geschlossenes Ende (16) und ein entgegengesetzt liegendes offenes Ende mit einer Kupplungseinrichtung (36) aufweist, die es in abgedichteter Beziehung mit der ersten Kapselsektion verbindet,
ein zylindrisches Gewicht (19) und eine Feder (21), die in der zweiten Kaspel angeordnet sind,
eine feste Medikamentenmatrix von zylindrischer Gestalt (17), die im Innenraum der ersten Kapselsektion angeordnet ist und durch Pansenflüssigkeit auflösbar oder dispergierbar ist,
wobei die Feder so vorgespannt ist, daß sie das ferne Ende (32) der Medikamentenmatrix gegen die Medikamentenmatrixhalteeinrichtung und in Richtung auf das erste offene Ende der ersten Kapselsektion drückt, dadurch genennzeichnet, daß
das Gewicht von der Feder umgeben ist, die von wendelförmiger Gestalt ist.

2. Medikamentenabgabevorrichtung nach Anspruch 1, bei der die Medikamentenmatrixhaltevorrichtung (34) einen konvergierenden ringförmigen Flansch aufweist, der die erste Öffnung der ersten Sektion ausbildet.

3. Medikamentenabgabevorrichtung nach Anspruch 2, bei der die feste Medikamentenmatrix von der zylindrischen Wand der ersten Kapselsektion eng umschlossen wird.

4. Medikamentenabgabevorrichtung nach Anspruch 3, bei der das Gewicht innerhalb der Kapsel festgehalten ist.

5. Medikamentenabgabevorrichtung nach Anspruch 4, bei der ein Ende des Gewichts in enger Passung mit der zylindrischen Wand der zweiten Kapselsektion ist.

6. Medikamentenabgabevorrichtung nach Anspruch 3, bei der das zylindrische Gewicht einen Umfangsabschnitt (25) aufweist, der mit einer inneren Halteeinrichtung (27) in der Kapsel in Eingriff ist.

7. Medikamentenabgabevorrichtung nach Anspruch 6, bei der die zweite Sektion der Kapsel einen ringförmigen Haltering (27) aufweist, der in einer ringförmigen Rille (25) des zylindrischen Gewichts sitzt.

8. Medikamentenabgabevorrichtung nach Anspruch 7, bei der ein Kolben (23) zwischen der Medikamentenmatrix und dem benachbarten Ende (22) der Feder angeordnet ist, wobei sich eine zylindrische Seitenwand über das benachbarte Federende und in dicht gleitendem Kontakt mit der Innenwand der Kapsel befindet.

9. Medikamentenabgabevorrichtung nach Anspruch 8, bei der der Kolben einen sich nach außen erstreckenden, schrägen Flansch (29) in dichter Anlage an der zylindrischen Wand der ersten Sektion aufweist und wobei diese Sektion ein Belüftungsloch aufweist.

**Revendications**

1. Dispositif distributeur de médicament destiné à être utilisé dans le réticulorumen d'un animal, ce dispositif comprenant:

une capsule cylindrique (11) constituée d'une matière plastique inerte, cette capsule ayant une première (13) et une seconde (15) section creuses,

cette première section de la capsule étant d'une configuration cylindrique allongée avec une première extrémité ouverte (33) et comportant un moyen de retenue (34) de la matrice de médicament,

cette première section ayant une seconde extrémité ouverte opposée à la première et réalisée avec un moyen d'accouplement (35),

cette seconde section de la capsule ayant une extrémité fermée (16) et une extrémité ouverte opposée avec un moyen d'accouplement (36) la raccordant en relation d'étanchement à la première section de la capsule,

un poids cylindrique (19) et un ressort (21) situés à l'intérieur de la seconde capsule,

une matrice de médicament (17) solide dispersable ou pouvant être dissoute par le fluide du rumen, cette matrice ayant une configuration cylindrique, tandis qu'elle est située à l'intérieur de la première section de la capsule,

ce ressort étant poussé pour amener l'extrémité distale (32) de la matrice de médicament contre le moyen de retenue de celle-ci et vers la première extrémité ouverte de la première section de la capsule, caractérisé en ce que ce poids est entouré par le ressort qui est d'une configuration hélicoïdale.

2. Dispositif distributeur de médicament selon la revendication 1, caractérisé en ce que le moyen de retenue (34) de la matrice de médicament comprend un rebord annulaire convergent définissant cette première ouverture de la première section.

3. Dispositif distributeur de médicament selon la revendication 2, caractérisé en ce que la matrice de médicament solide est contenue de manière bien ajustée dans la paroi cylindrique de la première section de la capsule.

4. Dispositif distributeur de médicament selon la revendication 3, caractérisé en ce que le poids précité est immobilisé à l'intérieur de la capsule.

5. Dispositif distributeur de médicament selon la revendication 4, caractérisé en ce qu'une extrémité du poids précité vient s'adapter avec un adjustage serré sur la paroi cylindrique de la seconde section de la capsule.

6. Dispositif distributeur de médicament selon la revendication 3, caractérisé en ce que le poids cylindrique a une partie circonférentielle (20) venant s'engager sur un moyen de retenue intérieur (25) prévu dans la capsule.

7n25) prévu dans la capsule.

7. Dispositif distributeur de médicament selon la revendication 6, caractérisé en ce que la seconde section de la capsule comporte une bague annulaire de reteue (27) venant prendre appui dans une gorge annulaire (25) du poids cylindrique.

8. Dispositif distributeur de médicament selon la revendication 7, caractérisé en ce qu'un piston (23) est placé entre la matrice de médicament et l'extrémité adjacente (22) du ressort, une paroi latérale cylindrique s'étendant par-dessus cette extrémité adjacente du ressort et en contact glissant étroit avec la paroi intérieure de la capsule.

9. Dispositif distributeur de médicament selon la revendication 8, caractérisé en ce que le piston comporte un rebord conique s'étendant vers l'extérieur (29) en relation d'étanchement avec la paroi cylindrique de la première section, cette section comportant un trou d'évent.

FIG. 1

FIG. 2

FIG. 3

FIG. 4

FIG. 5